# EUROPÄISCHE PATENTANMELDUNG

(11) **EP 4 846 950 A1**
(43) Veröffentlichungstag der Anmeldung: **02.12.2026**
(21) Anmeldenummer: 26162633.7
(22) Anmeldetag: 05.03.2026
(51) Int. Cl.: A61B 17/16, A61B 17/29

(54) **CHIRURGISCHES INSTRUMENT**

(30) Priorität: 26.05.2025 DE 102025120359
(71) Anmelder: Tontarra Medizintechnik GmbH, 78573 Wurmlingen (DE)
(72) Erfinder: TONTARRA, Thomas, 78573 Wurmlingen (DE); NEUBRAND, Florian, 78194 Immendingen (DE); TRUCKENBROD, Stefan, 78576 Emmingen-Liptingen (DE)
(74) Vertreter: Mammel und Maser Patentanwälte PartG mbB

(57) **Zusammenfassung**

Die Erfindung betrifft ein chirurgisches Instrument (11), mit einem Schaft (12), mit einem Handgriff (19) und mit einem dazwischen angeordneten Funktionsabschnitt (41), wobei in dem Funktionsabschnitt (41) eine Rückstelleinrichtung (51) vorgesehen ist und in dem Funktionsabschnitt (41) zumindest eine Spülöffnung (61) ausgebildet ist.

## Beschreibung

Die Erfindung betrifft ein chirurgisches Instrument, insbesondere Schiebeschaftinstrument.

Aus der EP 2 314 234 A1 ist ein chirurgisches Instrument, insbesondere Schiebeschaftinstrument, bekannt. Dieses chirurgische Instrument umfasst ein Hauptteil und ein relativ dazu bewegbares Teil. An dem Hauptteil ist ein Handgriff angeordnet, der einen feststehenden Griff und einen das bewegbare Teil betätigbaren Griff aufweist. Des Weiteren ist eine Führung vorgesehen, durch welche das bewegbare Teil relativ zum Hauptteil zwischen einer Ausgangsposition und einer Arbeitsposition bewegbar ist. Das bewegbare Teil weist eine Gleitfläche auf, die zum Hauptteil gerichtet ist und an einer Führungsfläche an dem Hauptteil verschiebbar geführt ist. Die Gleitfläche oder die Führungsfläche sind zur Bildung eines spülbaren Spaltes einander zugeordnet und in der Breite reduziert. An dem Handgriff ist zwischen dem feststehenden Griff und dem betätigbaren Teil ein Federelement vorgesehen, durch welches der betätigte Griff in eine Ausgangsposition zurückgeführt wird oder in dieser gehalten ist. Zur Reinigung dieses chirurgischen Instruments ist eine Zerlegung bzw. ein Entfernen des bewegbaren Teils von dem Hauptteil nicht erforderlich.

Aus der EP 4 309 594 B1 ist eine zerlegbare Knochenstanze bekannt. Bei dieser Knochenstanze ist vorgesehen, dass ein bewegbares Schaftteil ausgehend von einer Ausgangsposition in eine Zerlegeposition übergeführt wird, um dieses darauffolgend von dem feststehenden Schaftteil abzunehmen. Diese Knochenstanze weist ein inneres Rückstellelement auf, durch welches das betätigbare Griffteil aus einer Arbeitsposition in eine neutrale Position bzw. Ausgangsposition übergeführt wird. Nach dem Überführen des betätigbaren Griffteils in die Zerlegeposition und dem Abnehmen des bewegbaren Schaftteils wird auch das innere Rückstellelement aus dem Hauptteil zur Reinigung entnommen. Dadurch ist der zeitliche Aufwand für die Reinigung einer solchen Knochenstanze sehr aufwendig, insbesondere aufgrund dem aufeinanderfolgenden Einsetzen von dem Rückstellelement und dem bewegbaren Schaftteil zum feststehenden Schaftteil nach der Reinigung.

Der Erfindung liegt die Aufgabe zugrunde, ein chirurgisches Instrument vorzuschlagen, bei dem eine einfache Reinigung mit einem innenliegenden Rückstellelement für den Handgriff ermöglicht ist.

Diese Aufgabe wird durch ein chirurgisches Instrument, insbesondere Schiebeschaftinstrument, mit den Merkmalen des Anspruchs 1 gelöst.

Dieses chirurgische Instrument umfasst einen Schaft, der ein erstes Schaftteil und ein dazu bewegbares zweites Schaftteil aufweist, wobei jedes Schaftteil zumindest ein Führungselement umfasst, die verschiebbar miteinander verbunden sind. Des Weiteren ist ein Handgriff vorgesehen, wobei der Handgriff ein feststehendes Griffteil und ein betätigbares Griffteil aufweist, welches um eine Drehachse an dem feststehenden Griffteil mit dem ersten Schaftteil fest verbunden ist. Durch den betätigbaren Griff ist das zweite Schaftteil zwischen einer Ausgangsposition und einer Arbeitsposition verschiebbar. Des Weiteren ist eine Rückstelleinrichtung vorgesehen, durch welche der Handgriff in der Ausgangsposition gehalten ist oder aus der Arbeitsposition in die Ausgangsposition zurücksetzbar ist. Die innere oder innenliegende Rückstelleinrichtung umfasst zumindest ein Kraftspeicherelement, welches in einem Aufnahmeraum eines Funktionsabschnittes zwischen dem feststehenden Handgriff und dem feststehenden Schaftteil angeordnet ist. Ein Steckteil des betätigbaren Griffteils greift an der Rückstelleinrichtung an. An bzw. in dem Funktionsabschnitt, der zwischen dem feststehenden Griffteil und dem feststehenden Schaft ausgebildet ist, ist zumindest eine Spülöffnung vorgesehen, durch welche die innenliegende Rückstelleinrichtung von außen zugänglich ist. Diese zumindest eine Spülöffnung ermöglicht, dass ein Zerlegen des chirurgischen Instrumentes zur Reinigung nach dem chirurgischen Eingriff nicht erforderlich ist. Vielmehr wird durch die zumindest eine Spülöffnung ermöglicht, dass die innenliegende Rückstelleinrichtung in dem Funktionsabschnitt verbleibt, gereinigt und anschließend sterilisiert werden kann. Dies ermöglicht eine Vereinfachung bei der Reinigung solcher chirurgischen Instrumente sowie eine erhebliche Zeitreduzierung, da ein aufwendiger Zusammenbau eines zerlegten chirurgischen Instrumentes nicht mehr erforderlich ist. Somit ist eine nicht zerlegbare Knochenstanze geschaffen, welche eine einfache Reinigung bei einer innenliegenden Rückstelleinrichtung ermöglicht.

Bevorzugt ist vorgesehen, dass die zumindest eine Spülöffnung an einer Seitenwange des Funktionsabschnitts ausgebildet ist. Diese Seitenwange erstreckt sich entlang einer Längsachse des Schaftes. Dadurch ist eine seitliche Zuführung eines Reinigungsmediums in den Aufnahmeraum des Funktionsabschnitts ermöglicht.

Des Weiteren ist bevorzugt vorgesehen, dass die Spülöffnung als eine längliche Öffnung oder Langlochöffnung oder aus mehreren aneinandergereihten Einzelöffnungen ausgebildet ist. Insbesondere ist an beiden einander gegenüberliegenden Seitenwangen jeweils zumindest eine Spülöffnung vorgesehen. Bevorzugt sind die einander gegenüberliegenden Spülöffnungen zueinander ausgerichtet, insbesondere deckungsgleich zueinander ausgerichtet.

Der Aufnahmeraum in dem Funktionsabschnitt weist bevorzugt eine distale Öffnung auf, in welche die Rückstelleinrichtung einsetzbar ist. Dies ermöglicht zum einen eine einmalig einfache Montage. Zum anderen kann durch diese distale Öffnung eine weitere Spülöffnung ausgebildet sein, die in Verbindung mit der zumindest einen Spülöffnung an einer oder beiden Seitenwangen im Funktionsabschnitt zumindest einen Durchgangskanal bilden kann.

Der Aufnahmeraum in dem Funktionsabschnitt ist bevorzugt als eine Bohrung ausgebildet, insbesondere als eine Sacklochbohrung, in welche die Rückstelleinrichtung einsetzbar ist. Zwischen der Rückstelleinrichtung und der distalen Öffnung des Aufnahmeraumes ist ein Steckteil des betätigbaren Griffteils vorgesehen, durch welches die Rückstelleinrichtung in dem Aufnahmeraum fixiert gehalten ist und/oder das betätigbare Griffteil in die Ausgangsposition übergeführt oder gehalten wird.

Bevorzugt ist vorgesehen, dass der Aufnahmeraum an einem proximalen Endbereich zumindest eine weitere Spülöffnung aufweist. Dadurch kann eine verbesserte Reinigung des Aufnahmeraums und der innenliegenden Komponenten ermöglicht werden.

Die Rückstelleinrichtung des chirurgischen Instrumentes umfasst zumindest eine Rückstellfeder und ein Druckstück, wobei ein Druckstückkopf des Druckstücks an dem Steckteil anliegend in dem Aufnahmeraum angeordnet ist. Dadurch ist eine langlebige Ausführungsform von einer Rückstelleinrichtung geschaffen.

Die zumindest eine Spülöffnung an einer Seitenwange des Funktionsabschnitts erstreckt sich bevorzugt zumindest über eine Länge, sodass die Rückstelleinrichtung zumindest teilweise von außen zugänglich ist und/oder ein Bereich des Steckteils des betätigbaren Griffteils zumindest teilweise von außen zugänglich ist.

Insbesondere ist vorgesehen, dass der Druckstückkopf des Druckstücks kegelstumpfförmig oder pilzkopfförmig ausgebildet ist und an dem Steckteil zum Druckstückkopf weisend eine gekrümmte oder kreisbogenförmige Steuernocke ausgebildet ist, durch welche bei einer Schwenkbewegung des betätigbaren Griffteils eine in der Längsachse der Rückstelleinrichtung ausgerichtete Kraft auf das Druckstück wirkt. Dadurch kann eine verkippungsfreie Krafteinleitung in die Rückstelleinrichtung ermöglicht sein.

Des Weiteren ist bevorzugt vorgesehen, dass jeweils oberhalb und unterhalb der Steuernocke an dem Steckteil des betätigbaren Griffteils eine Vertiefung vorgesehen ist. Dies ermöglicht sowohl bei der Anordnung des betätigbaren Griffs in der Ausgangsposition als auch in der Arbeitsposition einen vergrößerten Schwenkbereich des Steckteils. Des Weiteren kann eine Aufbauhöhe des Funktionsabschnitts zwischen einer Drehachse des betätigbaren Griffteils und dem bewegbaren Schaftteil verringert werden.

Vorteilhafterweise umfasst der Aufnahmeraum in dem Funktionsabschnitt zum bewegbaren Schaftteil weisend eine Durchbrechung. In der Durchbrechung ist ein distaler Anschlag für das Steckteil des betätigbaren Griffteils zur Positionierung des Griffteils in der Ausgangsposition gebildet. Dies ermöglicht in einfacher Weise die Ausbildung eines innenliegenden Anschlages.

Des Weiteren ist bevorzugt vorgesehen, dass die Durchbrechung in dem Aufnahmeraum am proximalen Ende einen weiteren Anschlag bildet, der bei einem geschlossenen Schneidwerkzeug nicht wirkt, jedoch eine weitere Schließbewegung des betätigbaren Griffs bei einer weiteren Schließkraft verhindert bzw. sperrt. Dadurch ist eine Überlastsicherung ausgebildet, durch welche eine Beschädigung der Schneidteile des Schneidwerkzeugs bei einer weiteren Betätigung des Handgriffs verhindert wird.

Nach einer weiteren bevorzugten Ausführungsform des chirurgischen Instrumentes ist vorgesehen, dass in dem bewegbaren Schaftteil insbesondere ein proximaler Bereich des bewegbaren Schafteils, zumindest eine weitere Spülöffnung, vorgesehen ist. Diese zumindest eine weitere Spülöffnung kann dem zumindest einen Führungselement zwischen dem bewegbaren Schaftteil und dem feststehenden Schaftteil zugeordnet sein. Diese zumindest eine Spülöffnung kann des Weiteren einem Auswerfer zugeordnet sein, der zum Schneidteil des bewegbaren Schaftteils ausgerichtet ist. Die zumindest eine Spülöffnung kann auch dem zumindest einen Führungselement und dem Auswerfer zugeordnet sein. Dies ermöglicht, dass auch in dem Bereich der Führungselemente und/oder in dem Bereich, in dem der Auswerfer den Führungselementen zugeordnet ist, eine gute Zugänglichkeit für das Reinigungsmedium und Spülbarkeit ermöglicht ist.

Des Weiteren ist bevorzugt vorgesehen, dass am proximalen Ende des bewegbaren Schaftteils der Auswerfer vorgesehen ist, der in der Ausgangsposition des bewegbaren Schaftteils gegenüber dem Schneidteil an dem bewegbaren Schaftteil in proximaler Richtung hervorsteht.

Der Auswerfer ist bevorzugt als ein separates Bauteil, insbesondere stiftförmig, ausgebildet, und an dem feststehenden Schaftteil fixiert gehalten. Dies ermöglicht einen konstruktiv einfachen Aufbau für den Auswerfer, ohne dass hohe Herstellkosten erforderlich sind.

Des Weiteren ist bevorzugt vorgesehen, dass sich der Auswerfer ausgehend von dem Schneidteil des bewegbaren Schaftteils, das in einer Ausgangsposition angeordnet ist, in proximaler Richtung über das erste Führungselement hinaus erstreckt und im Anschluss daran an dem feststehenden Schaftteil gehalten ist. Dies ermöglicht, dass die Schneid- und Spülfunktion in vollem Umfang aufrechterhalten bleibt.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
- Figur 1: eine schematische Seitenansicht eines chirurgischen Instruments,
- Figur 2: eine schematisch vergrößerte Seitenansicht eines Funktionsabschnitts des chirurgischen Instruments in einer Ausgangsposition gemäß Figur 1,
- Figur 3: eine schematische Schnittansicht des Funktionsabschnitts gemäß Figur 2,
- Figur 4: eine schematisch vergrößerte Seitenansicht des Funktionsabschnitts des chirurgischen Instruments in einer Arbeitsposition,
- Figur 5: eine schematische Schnittansicht des Funktionsabschnitts gemäß Figur 4,
- Figur 6: eine schematisch vergrößerte Seitenansicht von einem proximalen Ende des Schaftes des chirurgischen Instruments gemäß Figur 1,
- Figur 7: eine schematische Schnittansicht des Schaftes gemäß Figur 6,
- Figur 8: eine schematische Seitenansicht von einem proximalen Ende des Schaftes des chirurgischen Instrumentes in einer Arbeitsposition, und
- Figur 9: eine schematische Schnittansicht des Schaftes gemäß Figur 8.

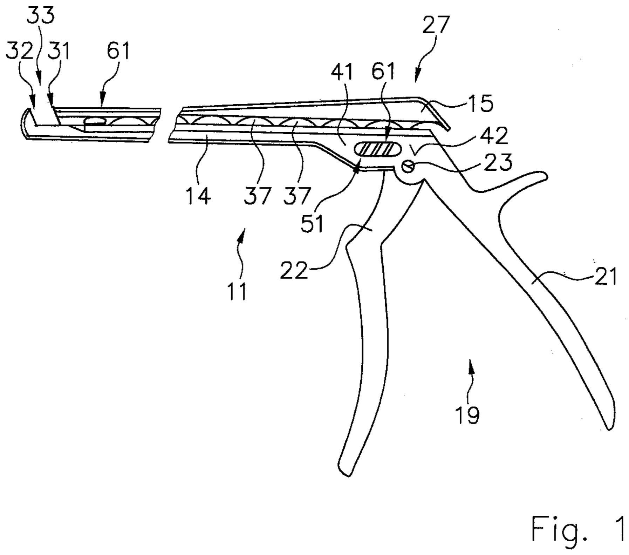

In Figur 1 ist eine schematische Seitenansicht eines chirurgischen Instruments 11 dargestellt. Bei diesem chirurgischen Instrument 11 handelt es sich beispielsweise um ein Schiebeschaftinstrument, eine sogenannte Stanze, insbesondere Kerrison-Stanze, welche bei chirurgischen Eingriffen für die Entfernung von Gewebe und/oder Knochen oder dergleichen eingesetzt wird. Die Erfindung ist nicht auf diese Stanzen beschränkt, sondern kann auf alle chirurgischen Instrumente übertragen werden, welche dieselbe Problematik hinsichtlich der Reinigung und Sterilisation aufweisen.

Das chirurgische Element 11 umfasst einen Schaft 12, wobei der Schaft 12 ein erstes Schaftteil 14 und ein dazu bewegbares Schaftteil 15 aufweist. Jedes Schaftteil 14, 15 weist zumindest ein Führungselement 16, 17 auf, die miteinander verbunden sind, um das bewegbare Schaftteil 15 an dem ersten Schaftteil 14 bewegbar zu führen.

Das chirurgische Instrument 11 umfasst des Weiteren einen Handgriff 19, welcher aus einem feststehenden Griffteil 21 und einem betätigbaren Griffteil 22 gebildet ist. Das betätigbare Griffteil 22 ist um eine Drehachse 23 mit dem feststehenden Griffteil 21 schwenkbar verbunden. Durch das betätigbare Griffteil 22 ist das bewegbare Schaftteil 15 relativ zum ersten Schaftteil 14 verschiebbar.

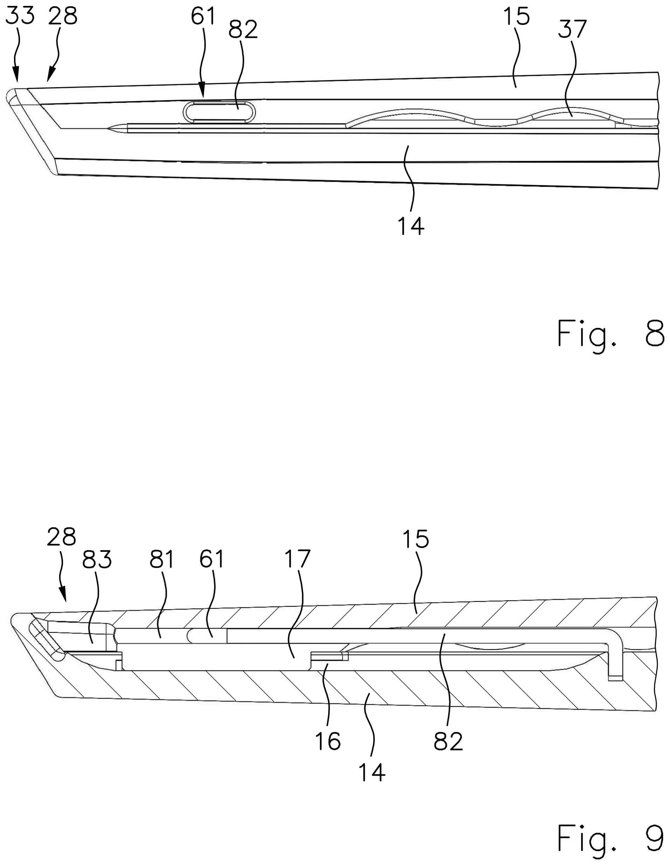

Das chirurgische Instrument 11 umfasst des Weiteren eine Rückstelleinrichtung 51, durch welche der Handgriff 19 in einer Ausgangsposition 27 gehalten ist oder aus einer Arbeitsposition 28 (Figur 8) in die Ausgangsposition 27 zurückführbar ist. Das chirurgische Instrument 11 ist gemäß Figur 1 in einer Ausgangsposition 27 dargestellt. In dieser Ausgangsposition 27 ist der Handgriff 19 aufgespreizt. Ein Schneidteil 31 eines Schneidwerkzeugs 33 des chirurgischen Instruments 11 ist an dem proximalen Ende des bewegbaren Schaftteils 15 mit Abstand zu einem Schneidteil 32 an dem ersten Schaftteil bzw. feststehenden Schaftteil 14 angeordnet. Die Ausgestaltung der Schneidteile 31, 32 ist nur beispielhaft. Anwendungsspezifisch können alternative Funktionselemente anstelle der Schneidteile 31, 32 jeweils am proximalen Ende des feststehenden Schaftteils 14 und des bewegbares Schaftteiles 15 vorgesehen sein. Das bewegbare Schaftteil 15 weist bevorzugt eine zum feststehenden Schaftteil 14 weisende Gleitfläche 34 auf, die entlang dem feststehenden Schaftteil 21 auf einer Führungsfläche 35 verschiebbar geführt ist. Die Gleitfläche 34 und/oder die Führungsfläche 35 sind zur Bildung eines spülbaren Spaltes 36 zwischen den beiden Flächen einander zugeordnet und ausgebildet. Bevorzugt ist die Gleitfläche 34 und/oder die Führungsfläche 35 in einer Raumrichtung durch zumindest eine Aussparung 37 durchbrochen. Hinsichtlich der möglichen Ausführungsformen wird vollumfänglich auf die EP 2 314 234 A1 Bezug genommen.

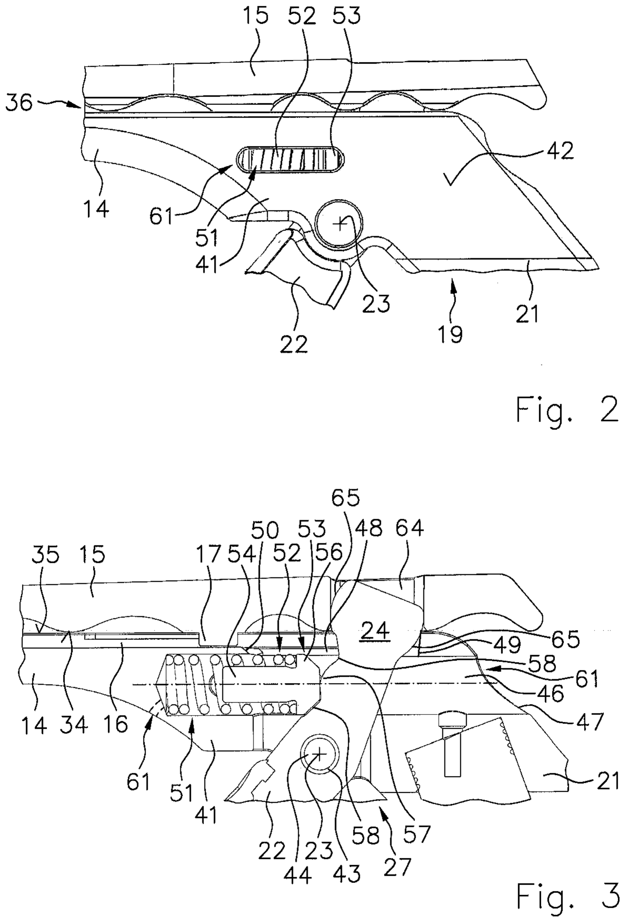

Das chirurgische Instrument 11 weist des Weiteren zwischen dem Handgriff 19 und dem ersten Schaftteil 14 einen Funktionsabschnitt 41 auf. Dieser Funktionsabschnitt 41 ist in Figur 2 in einer Seitenansicht vergrößert dargestellt. In Figur 3 ist eine Schnittansicht des Funktionsabschnittes 41 dargestellt. Der Handgriff 19 bzw. das bewegbare Schaftteil 15 sind in der Ausgangsposition 27 angeordnet und dargestellt.

Der Funktionsabschnitt 41 weist beidseitig jeweils eine Seitenwange 42 auf, die vorzugsweise parallel zueinander ausgerichtet sind. Diese Seitenwange 42 erstreckt sich zwischen dem feststehenden Griffteil 21 und dem feststehenden Schaftteil 15. Die Drehachse 23 ist rechtwinklig zu den Seitenwange 42 ausgerichtet. In dieser Drehachse 23 sind Verbindungselemente 43, 44 vorgesehen, durch welche das betätigbare Griffteil 22 um die Drehachse 23 schwenkbar gelagert ist. Sofern die Verbindungselemente 43, 44 gelöst werden, kann das betätigbare Griffteil 22 gegenüber dem feststehenden Griffteil 21 entfernt werden.

Das betätigbare Griffteil 22 erstreckt sich mit einem Griffbereich von der Drehachse 21 aus zu einem freien Ende. Gegenüberliegend ist ein Steckteil 24 ausgebildet, welches sich von der Drehachse 21 aus zumindest bis in das bewegbare Schaftteil 15 hinein erstreckt.

In dem Funktionsabschnitt 41 ist ein Aufnahmeraum 46 ausgebildet, der eine distale Öffnung 47 aufweist, von der aus sich der Aufnahmeraum 46 in proximaler Richtung innerhalb des Funktionsabschnittes 41 erstreckt. Der Aufnahmeraum 46 ist bevorzugt als eine Bohrung, insbesondere eine Sacklochbohrung, ausgebildet. Das Steckteil 24 durchquert diesen Aufnahmeraum 46 vollständig. Hierfür ist eine Durchbrechung 48 an einer oberen Stirnseite des Funktionsabschnittes 41 und zum bewegbaren Schaftteil 15 weisend vorgesehen. Diese Durchbrechung 48 ist schlitzförmig ausgebildet, innerhalb der sich das Steckteil 24 erstreckt. An einem distalen Ende der Durchbrechung 48 ist ein erster Anschlag 49 vorgesehen, der das betätigbare Griffteil 22 in einer aufgespreizten Position zum feststehenden Griffteil 21 begrenzt. Dadurch wird die Ausgangsposition 27 des chirurgischen Instrumentes 11 eingenommen.

In dem Funktionsabschnitt 41, insbesondere in dem Aufnahmeraum 46, ist eine Rückstelleinrichtung 51 angeordnet. Diese Rückstelleinrichtung 51 umfasst zumindest ein Kraftspeicherelement 52. Dieses Kraftspeicherelement 52 stützt sich zum einen an einem proximalen Ende in dem Aufnahmeraum 46 ab und zum anderen kann dieses Kraftspeicherelement 52 an dem Steckteil 24 angreifen. Bevorzugt ist vorgesehen, dass die Rückstelleinrichtung 51 zusätzlich ein Druckstück 53 umfasst. Dieses Druckstück 53 weist einen Führungsstift 54 auf, der innerhalb einem als spiralförmige Druckfeder ausgebildeten Kraftspeicherelement 52 geführt ist. Das Druckstück 53 umfasst des Weiteren einen Druckstückkopf 56, der zum Steckteil 24 ausgerichtet ist. Das Steckteil 24 weist einen zum Druckstück 53 weisenden Steuernocken 57 auf. Benachbart zu dem Steuernocken 57 ist jeweils eine Vertiefung 58 ausgebildet. Diese Vertiefung 58 sowie der Abstand zwischen den beiden Vertiefungen zur Ausbildung des Steuernockens 57 sind auf die Geometrie des Druckstückkopfes 56 angepasst. Der Druckstückkopf 56 ist bevorzugt kegelstumpfförmig oder kreisbogenförmig ausgebildet. In der in Figur 3 dargestellten Anordnung der Rückstelleinrichtung 51 ist ersichtlich, dass die Kontur des Druckstückkopfes 56 und die Kontur des Steckteils 24 in Bezug auf den Steuernocken 57 und die benachbarte Vertiefung 58 aneinander angepasst sind. Dadurch kann bei einem geringeren Bauraum eine vergrößerte Schwenkbeweglichkeit erzielt werden.

Die Rückstelleinrichtung 51 ist innenliegend in dem Funktionsabschnitt 41 angeordnet. Zur Reinigung dieser Rückstelleinrichtung 51 nach einem chirurgischen Eingriff wird das chirurgische Element 11 nicht zerlegt. Vielmehr ist an dem Funktionsabschnitt 41 zumindest eine Spülöffnung 61 vorgesehen. Durch diese Spülöffnung 61 ist ein freier Zugang eines Reinigungsmediums von außen zu der in dem Funktionsabschnitt 41 liegenden bzw. angeordneten Rückstelleinrichtung 51 gegeben. Zusätzlich ist ein freier Zugang des Reinigungsmediums über die Öffnung 47 in den Aufnahmeraum 46 gegeben. Des Weiteren kann bevorzugt vorgesehen sein, dass im proximalen Endbereich des Aufnahmeraums 46 zumindest eine weitere Spülöffnung 61 vorgesehen ist.

Die Spülöffnung 61 ist beispielsweise als eine Langlochbohrung ausgebildet. Die Geometrie und/oder die Kontur der Spülöffnung 61 kann beliebig gewählt werden. Eine Beschränkung ist dahingehend gegeben, dass eine sichere Aufnahme der Rückstelleinrichtung 51 und ein Verbleib der Rückstelleinrichtung 51 in dem Funktionsabschnitt 41 gewährleistet ist. Anstelle einer einzigen Spülöffnung 61 an einer Seitenwange 42 des Führungsabschnitts 41 können auch mehrere aneinandergereihte Spülöffnungen 61 mit gleicher und/oder voneinander abweichender Geometrie ausgebildet sein. Bevorzugt sind die Spülöffnungen 61 einander gegenüberliegend an der jeweiligen Seitenwange 42 ausgerichtet. Dadurch kann ein erhöhter Wirkungsgrad bei der Reinigung erzielt werden.

Die Länge von beispielsweise einer Spülöffnung 61 kann sich zumindest über die halbe Länge der Rückstelleinrichtung 51 und zumindest einem Abschnitt des Steckteils 24 erstrecken.

Das Steckteil 24 des betätigbaren Griffteils 22 ist in einer Führungsöffnung 64 im bewegbaren Schaftteil 15 positioniert. Diese Führungsöffnung 64 erstreckt sich in vorteilhafter Weise über die gesamte Höhe des bewegbaren Schaftteils 15 und kann somit in Verbindung mit der Durchbrechung 48 in dem Funktionsabschnitt 41 eine weitere Spülöffnung 61 bilden. Das Steckteil 24 weist bevorzugt gekrümmte oder kreisbogenförmige Führungsflächen 65 auf, sodass durch die Schwenkbewegung des Steckteils 24 um die Drehachse 23 eine Verschiebebewegung auf das bewegbare Schaftteil 15 übertragbar ist.

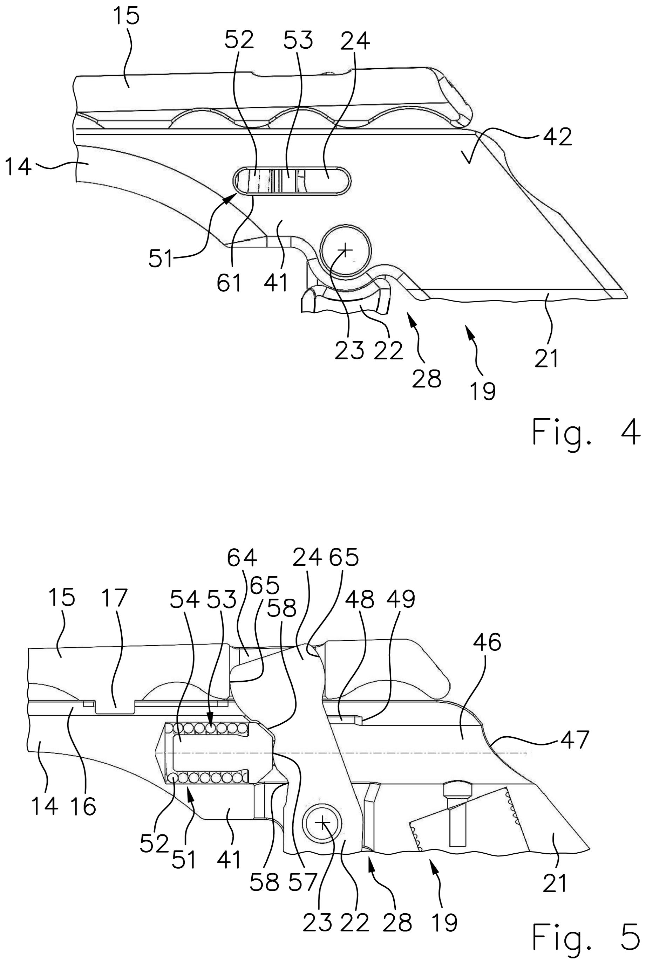

In Figur 4 ist eine schematisch vergrößerte Ansicht des Funktionsabschnittes 41 in Analogie zu Figur 2 dargestellt, wobei in Figur 4 das chirurgische Instrument 11 in eine Arbeitsposition 28 übergeführt ist. Die Figur 5 zeigt eine Schnittansicht des Funktionsabschnittes 41, um die veränderte Positionierung von dem betätigbaren Griffteil 22 und der Rückstelleinrichtung 51 zu erkennen. Das betätigbare Griffteil 22 ist auf das feststehende Griffteil 21 zubewegt, sodass das Schneidteil 31 an dem Schneidteil 32 anliegt und das Schneidwerkzeug 33 geschlossen ist.

Bei dieser Position des betätigbaren Griffteils 22 wird über den Steuernocken 57 das Druckstück 53 in eine proximale Richtung verschoben und das Kraftspeicherelement 52 zusammengedrückt. Während einer Schwenkbewegung des betätigbaren Griffteils 22 aus der Ausgangsposition 27 in die Arbeitsposition 28 wirkt der Steuernocken 57 an dem Steckteil 24 immer entlang einer Längsachse des Aufnahmeraumes 46 auf das Druckstück 53.

In der Arbeitsposition 28 kann sich wiederum der Druckstückkopf 56 in Richtung der Vertiefung 58 benachbart zum Steuernocken 57 bewegen. Dabei wird dieselbe Funktionsweise und der Vorteil erzielt, wie dies in Figur 3 beschrieben ist.

Die Durchbrechung 48 im Funktionsabschnitt 41 weist dem ersten Anschlag 49 gegenüberliegend einen zweiten Anschlag 50 auf. Durch die beiden Anschläge 49, 50 ist die Länge der Durchbrechung 48 begrenzt. In der Arbeitsposition 28 des betätigbaren Griffteils 22 liegt ein proximaler Bereich des Steckteils 24 bzw. eine gekrümmte Führungsfläche 65 des Steckteils 24 gerade nicht an dem Anschlag 50 an. Das Schneidwerkzeug 33 ist hingegen geschlossen. Bei einem gegebenenfalls weiteren Aufbringen einer Schließkraft auf das betätigbare Griffteil 22 wird die weitere Drehbewegung des Steckteils 24 durch den Anschlag 50 begrenzt, sodass eine Überlast auf das Schneidwerkzeug 33 vermieden wird.

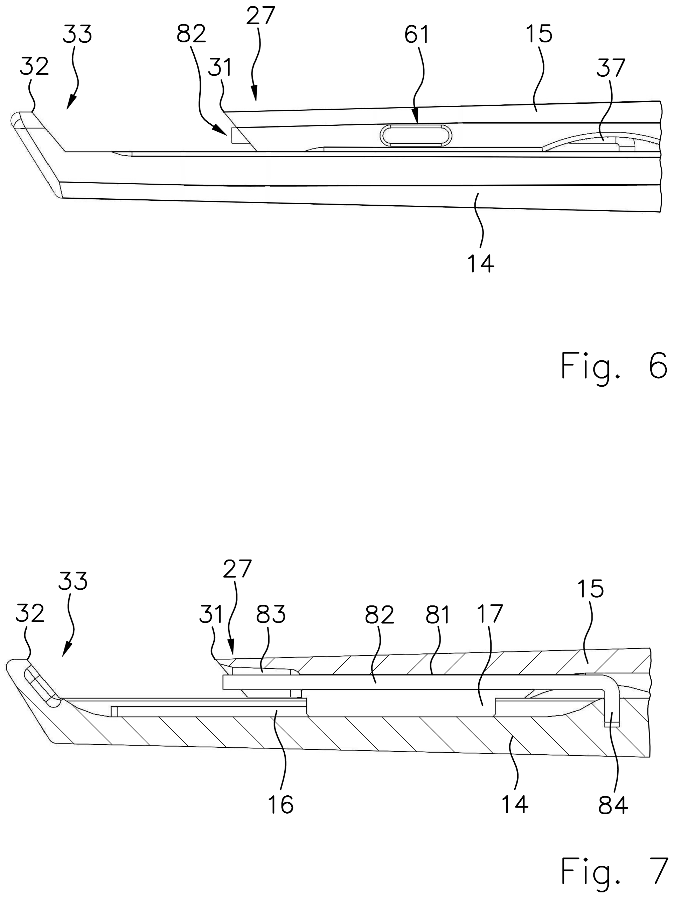

In Figur 6 ist eine schematische Seitenansicht des Schneidwerkzeuges 33 in einer geöffneten Position des chirurgischen Instruments 11 dargestellt. In Figur 7 ist das Schneidwerkzeug 33 gemäß Figur 6 in einer Schnittansicht dargestellt.

Das bewegbare Schaftteil 15 ist benachbart zum Schneidteil 31 mit dem Führungselement 17 an dem Führungselement 16 des ersten Schaftteils 14 geführt. Am proximalen Ende des bewegbaren Schaftteils 15 ist des Weiteren eine Durchgangsbohrung 81 vorgesehen, welche zur Aufnahme eines Auswerfers 82 dient. Diese Durchgangsbohrung 81 erstreckt sich bevorzugt oberhalb des Führungselements 16. Der Auswerfer 82 ist stiftförmig ausgebildet und ragt in der Ausgangsposition 27 des bewegbaren Schaftteils 15 gegenüber dem Schneidteil 31 hervor. Gegenüberliegend weist der Auswerfer 82 einen Befestigungsabschnitt 84 auf. Dieser kann beispielsweise durch einen abgewinkelten Endbereich von dem stiftförmigen Auswerfer 82 ausgebildet sein. Dieser abgewinkelte Endbereich kann in einer Bohrung am ersten Schaftteil 14 eingehakt bzw. gesichert werden.

Das Schneidteil 31 kann beispielsweise als ein U-förmige Schneide ausgebildet sein. An das Schneidteil 31 angrenzend und in distale Richtung gesehen ist an dem bewegbaren Schaftteil 15 ein Freiraum 83 vorgesehen, in welchem sich beim Schneiden von einem Gewebe und/oder Knochen ansammeln können. Sofern das bewegbares Schaftteil 15 aus der Arbeitsposition 28 in die Ausgangsposition 27 übergeführt wird, erfolgt mittels dem Auswerfer 82 ein Ausstoßen des in dem Freiraum 83 aufgenommenen Gewebes und/oder Knochen.

An dem bewegbaren Schaftteil 15 ist in dem Bereich des Führungselementes 17 und/oder in dem Bereich des Auswerfers 82 zumindest eine weitere Spülöffnung 61 vorgesehen. Dies ermöglicht, dass sowohl die Führungselemente 16, 17 und/oder die Durchgangsbohrung 81 mit einem Reinigungsmedium gespült werden können. Vorteilhafterweise ist an jeder Außenseite des bewegbaren Schaftteils 15 jeweils zumindest eine Spülöffnung 61 vorgesehen. Im Hinblick auf die Ausführungsformen der Spülöffnung 61 kann auf die Ausführungen zu dem Funktionsabschnitt 42 Bezug genommen werden.

In Figur 8 ist das Schneidwerkzeug 33 in einer Arbeitsposition 28 des chirurgischen Instruments 11 dargestellt. Die Figur 9 zeigt eine Schnittansicht der Figur 8.

In dieser Arbeitsposition 28 ist das Schneidwerkzeug 33 geschlossen. Das Schneidteil 31 liegt an dem Schneidteil 32 zum Trennen von einem Gewebe und/oder einem Knochen oder dergleichen an. Die Spülöffnung 61 ist beispielsweise derart positioniert, dass ein proximales Ende des Auswerfers 82 im Bereich der Spülöffnung 61 liegt.

## Patentansprüche

1. Chirurgisches Instrument, insbesondere Schiebeschaftinstrument,
- mit einem Schaft (12), der ein erstes Schaftteil (14) und ein bewegbares Schaftteil (15) umfasst, wobei jedes Schaftteil (14, 15) zumindest ein Führungselement (16, 17) aufweist, die miteinander verschiebbar verbunden sind,
- mit einem Handgriff (19), der ein feststehendes Griffteil (21) und ein betätigbares Griffteil (22) umfasst, wobei das betätigbare Griffteil (22) um eine Drehachse (23) schwenkbar ist und das betätigbare Griffteil (22) mit dem bewegbaren Schaftteil (15) gelenkig verbunden ist, wobei das bewegbare Schaftteil (15) durch das betätigbare Griffteil (22) aus einer Ausgangsposition (27), in welcher ein Schneidteil (31) des bewegbaren Schaftteils (15) mit Abstand zu einem Schneidteil (32) an dem ersten Schaftteil (14) angeordnet ist, in einer Arbeitsposition (28), in welcher die Schneidteile (31, 32) aneinander liegen, überführbar ist,
- mit einer Rückstelleinrichtung (51), durch welche der Handgriff (19) in der Ausgangsposition (27) gehalten ist oder aus der Arbeitsposition (28) in die Ausgangsposition (27) zurücksetzbar ist, wobei die Rückstelleinrichtung (51) zumindest ein Kraftspeicherelement (52) in einem Aufnahmeraum (46) eines Funktionsabschnittes (41) aufweist, der zwischen dem feststehenden Griffteil (21) und dem feststehenden Schaftteil (14) gebildet ist, und
- mit einem Steckteil (24) an dem betätigbaren Griffteils (22), welches an der Rückstelleinrichtung (26) angreift,
**dadurch gekennzeichnet,**
- **dass** in dem Funktionsabschnitt (41) zumindest eine Spülöffnung (61) vorgesehen ist, durch welche die Rückstelleinrichtung (51) von außen zugänglich ist.

2. Chirurgisches Instrument nach Anspruch 1, **dadurch gekennzeichnet, dass** die zumindest eine Spülöffnung (61) an einer oder beiden Seitenwangen (42) des Funktionsabschnittes (41) angeordnet ist.

3. Chirurgisches Instrument nach Anspruch 1 oder 2, **dadurch gekennzeichnet, dass** die zumindest eine Spülöffnung (61) an der Seitenwange (42) des Funktionsabschnitts (41) zumindest eine Länge aufweist, sodass die Rückstelleinrichtung (51) und/oder ein Bereich des Steckteils (24) des betätigbaren Griffteils (22) zumindest teilweise von außen zugänglich ist.

4. Chirurgisches Instrument nach einem der vorhergehenden Ansprüche, **dadurch gekennzeichnet, dass** die zumindest eine Spülöffnung (61) als eine langlochförmige Öffnung ausgebildet ist, oder dass mehrere aneinandergereihte Einzelöffnungen ausgebildet sind.

5. Chirurgisches Instrument nach einem der vorhergehenden Ansprüche, **dadurch gekennzeichnet, dass** der Aufnahmeraum (46) in dem Funktionsabschnitt (41) eine distale Öffnung (47) aufweist, in welche die Rückstelleinrichtung (51) einsetzbar ist, und vorzugsweise der Aufnahmeraum (46) als eine Bohrung ausgebildet ist.

6. Chirurgisches Instrument nach einem der vorhergehenden Ansprüche, **dadurch gekennzeichnet, dass** der Aufnahmeraum (46) im Funktionsabschnitt (41) an einem proximalen Endbereich zumindest eine weitere Spülöffnung (61) aufweist.

7. Chirurgisches Instrument nach einem der vorhergehenden Ansprüche, **dadurch gekennzeichnet, dass** die Rückstelleinrichtung (51) zumindest das Kraftspeicherelement (52) und ein Druckstück (53) umfasst, wobei das Druckstück (53) einen Druckstückkopf (56) aufweist, der an dem Steckteil (24) anliegend in dem Aufnahmeraum (46) angeordnet ist.

8. Chirurgisches Instrument nach einem der vorhergehenden Ansprüche, **dadurch gekennzeichnet, dass** das Steckteil (24) des betätigbaren Griffteils (22) zum Druckstück (53) weisend eine gekrümmte oder kreisbogenförmige Steuernocke (57) aufweist, durch welche bei einer Schwenkbewegung des betätigbaren Griffteils (22) eine in einer Längsachse der Rückstelleinrichtung (51) ausgerichtete Kraft auf das Druckstück (53) wirkt.

9. Chirurgisches Instrument nach Anspruch 7 oder 8, **dadurch gekennzeichnet, dass** oberhalb und unterhalb der Steuernocke (57) an dem Steckteil (24) des betätigbaren Griffteils (22) eine Vertiefung (58) angeordnet ist.

10. Chirurgisches Instrument nach einem der vorgehenden Ansprüche, **dadurch gekennzeichnet, dass** zwischen dem Aufnahmeraum (46) und dem bewegbaren Schaftteil (15) eine Durchbrechung (48) in dem Funktionsabschnitt (41) angeordnet ist, durch welche sich das Steckteil (24) erstreckt, und ein distales Ende der Durchbrechung (48) einen ersten Anschlag (49) für das Steckteil (24) des betätigbaren Griffteils (22) zur Positionierung in der Ausgangsposition (27) bildet und vorzugsweise die Durchbrechung (48) am proximalen Ende einen weiteren Anschlag (50) bildet, der bei der Positionierung des bewegbaren Schaftteils (15) in der Arbeitsposition (28) nicht wirkt, jedoch eine weitere Schließbewegung des betätigbaren Griffs (22) sperrt.

11. Chirurgisches Instrument nach einem der vorhergehenden Ansprüche, **dadurch gekennzeichnet, dass** in dem bewegbaren Schaftteil (15) zumindest eine weitere Spülöffnung (61) vorgesehen ist.

12. Chirurgisches Instrument nach Anspruch 11, **dadurch gekennzeichnet, dass** die zumindest eine weitere Spülöffnung (61) dem zumindest einen Führungselement (16, 17) zugeordnet ist.

13. Chirurgisches Instrument nach Anspruch 11 oder 12, **dadurch gekennzeichnet, dass** die zumindest eine weitere Spülöffnung (61) einem Auswerfer (82) zugeordnet ist, der im proximalen Endbereich des bewegbaren Schaftteils (15) vorgesehen ist.

14. Chirurgisches Instrument nach Anspruch 13, **dadurch gekennzeichnet, dass** der Auswerfer (82) als ein separates Bauteil, insbesondere stiftförmig, ausgebildet ist und an dem feststehenden Schaftteil (14) fixiert gehalten ist.

15. Chirurgisches Instrument nach einem der Ansprüche 13 oder 14, **dadurch gekennzeichnet, dass** sich der Auswerfer (82) bei einer Anordnung des bewegbaren Schaftteils (15) in einer Ausgangsposition (27) in proximaler Richtung über das Schneidteil (31) an dem bewegbaren Schaftteil (15) hinaus erstreckt.
